Europäisches Patentamt

European Patent Office

Office européen des brevets

(11) Publication number: **0 242 154 B1**

## EUROPEAN PATENT SPECIFICATION

(45) Date of publication of patent specification: **03.03.93**

(21) Application number: **87303195.9**

(22) Date of filing: **13.04.87**

(51) Int. Cl.5: **C07K 15/06**, C12P 21/00, C12N 5/00, A61K 39/395, A61K 43/00, A61K 49/02, G01N 33/574, G01N 33/577, //C12N15/00,(C12P21/00, C12R1:91)

(54) A novel tumor-associated antigen.

(30) Priority: **17.04.86 US 853073**

(43) Date of publication of application:
**21.10.87 Bulletin  87/43**

(45) Publication of the grant of the patent:
**03.03.93 Bulletin  93/09**

(84) Designated Contracting States:
**AT BE CH DE ES FR GB GR IT LI LU NL SE**

(56) References cited:
**EP-A- 0 112 093**
**EP-A- 0 243 058**
**WO-A-85/03132**

(73) Proprietor: **HYBRITECH INCORPORATED**
**11095 Torreyana Road**
**San Diego California 92126(US)**

(72) Inventor: **Burnett, Karen Gray**
**10295 Rue Chamberry**
**San Diego California 92131(US)**
Inventor: **Grauer, Lana Suzanne**
**13252 Caminito Point Del Mar**
**Del Mar California 92014(US)**
Inventor: **Oh, Esther Hui-Yim**
**11117 Pegusus Avenue**
**San Diego California 92126(US)**

(74) Representative: **Hudson, Christopher Mark et al**
**Erl Wood Manor**
**Windlesham Surrey GU20 6PH (GB)**

BIOLOGICAL ABSTRACTS, vol. 79, 1985, abstract no. 106303, Biological Abstracts, Inc., Philadelphia, PA, US; J. KOVARIK et al.: "Biochemical and hitochemical characteristics of target antigen detected by monoclonal antibody HBCA-12 against a membrane component of human mammary carcinoma cell line"

FEDERATION PROCEEDINGS, 1986, vol. 45, no. 4, page 984, abstract no. 4830; E. OH et al.: "A breast cancer antigen targeted in vivo by a human monoclonal antibody"

PROCEEDINGS NATIONAL ACADEMY SCIENCES USA, vol. 79, September 1982, pages 5420-5424; R.L. CERIANI et al.: "Circulating human mammary epithelial antigens in breast cancer"

PROCEEDINGS NATIONAL ACAD. SCIENCES, USA, vol. 77, no. 11, November 1980, pages 6841-6845; J. SCHLOM et al.: "Generation of human monoclonal antibodies reactive with human mammary carcinoma cells"

## Description

The present invention relates to the characterization of antigens, particularly tumor-associated antigens. In another aspect, it relates to antibodies having specific reactivity with such antigens. In yet another aspect, it relates to methods for producing such antibodies and diagnostic and therapeutic uses therefor.

The potential role of monoclonal antibodies in the diagnosis and treatment of cancer in humans has been the focus of much recent investigation and speculation. Of particular interest is their use in immunoassays to detect and monitor the course of cancer, e.g., during therapy. Also of particular interest are the potential applications of monoclonal antibodies for tumor imaging and therapy due to their capacity to bind tumor-associated antigens in vivo.

Developments in monoclonal antibody technology have also made it possible to investigate the antigenic complexity of human tumors. Specifically, the precise immunoreactivity of monoclonal antibodies permits the identification and differentiation of antigens expressed by human tumors. The characterization, therefore, of such distinct tumor-associated antigens by their reactivity with certain monoclonal antibodies provides a means to exploit more fully the use of monoclonal antibody technology for cancer diagnosis and therapy.

Paradoxically, although certain antigens are expressed by human tumor cells, they are also expressed by normal cells. Accordingly, these antigens are referred to not as "tumor specific but as tumor-associated" antigens. The diagnostic and therapeutic value of such tumor-associated antigens results from the excess quantity of antigen expressed by tumor cells relative to normal cells and, in addition, the selectivity of antibodies in vivo for antigen expressed by tumor cells rather than normal cells. The relative selectivity of antibodies administered in vivo for antigen expressed by tumor cells is believed to result from: (1) the increased expression of antigen by cancerous cells due to the altered and rapid metabolism of malignant growth; and, (2) the increased accessability of antigens of tumor tissue to antibodies due to the breakdown of barrier properties of the membranes of tumor cells.

To date, only a limited number of tumor-associated antigens are well characterized. Additionally, certain tumor-associated antigens useful as diagnostic or prognostic markers may not be present in all patients or during all stages and manifestations of the disease. Diagnostic discrimination and therapeutic efficacy are, therefore, enhanced by the identification and characterization of more than one tumor-associated antigen expressed by the same tumor tissue. Further, for purposes of cancer diagnosis and therapy, it is desirable to rely on a set or panel of distinct antigens associated with specific types of human tumors. Accordingly, there exists a need for further identification and characterization of unique tumor-associated antigens.

The present invention is based upon the characterization of a novel antigen present in human tissue, particularly breast carcinoma. Accordingly, the invention is directed to a distinct tumor-associated antigen, which can be characterized at least by its reactivity with monoclonal antibody YBY088.

Also in accordance with the present invention, antibodies having specificity for the antigen of the invention and methods for the production of such antibodies are provided. Additionally, the invention is directed to the use of such antibodies to detect cancer and monitor the course of the disease state by immunohistochemical and immunoassay procedures. The invention is further directed to the use of such antibodies for the in vivo diagnosis and treatment of cancer in humans.

As indicated above, the present invention provides a novel tumor-associated antigen. In accordance with the invention, monoclonal antibody YBY088 is utilized for characterization of this previously undescribed antigen. Monoclonal antibody YBY088 is generated by a hybrid cell line which has been deposited (April 16, 1986) with the American Type Culture Collection (ATCC), 12301 Parklawn Drive, Rockville, MD 20852. Samples can be obtained by reference to the accession number HB9076 assigned to the cell line deposit.

The physiochemical and immunological properties of the antigen of the present invention, and particularly, its reactivity with monoclonal antibody YBY088, permit its characterization and differentiation from other antigens present in normal and tumor tissue. Accordingly, the identifying characteristics and properties of the antigen provided by the present invention are as follows:

(a) The antigen is present in human breast cell lines, specifically the T47D breast cell line (ATCC HTB 133).

(b) The antigen is a membrane component of breast carcinoma. Standard enzyme-linked immunoabsorbent binding assay procedures (ELISA) using monoclonal antibody YBY088 indicate the presence of the antigen in plasma membranes purified from breast carcinoma and the presence of the antigen, in relatively lower concentrations, in plasma membranes purified from normal breast and pancreas tissue. By comparison, YBY088 exhibits no appreciable reactivity with membranes purified from such benign tissues as colon, liver, kidney, lung, prostrate and bladder tissue.

3

(c) The antigen is produced in the cytosol of a variety of normal tissues and breast carcinoma. As shown by ELISA techniques, YBY088 is reactive with cytosol purified from such benign tissues as breast, colon, liver, kidney, lung, prostrate, brain and pancreas, as well as such tumor tissues as breast carcinoma, colon carcinoma and the mucosa of colon carcinoma.

(d) SDS - polyacrylamide gel electrophoresis (SDS-PAGE) and Western immunoblot analysis with SDS extracts of the human breast cell line T47D reveal that YBY088 reacts with molecular weight components of the antigen within the ranges of about 68,000 to about 80,000, about 40,000 to about 48,000, about 28,000 to about 32,000, and about 16,000 to about 19,000. Alternatively, SDS-PAGE and Western immunblot analysis with non-ionic detergent extracts of the human breast cell line T47D indicate that YBY088 reacts with molecular weight components of the antigen within the ranges of about 28,000 to about 32,000, and about 16,000 to about 19,000. Modification of the antigen by destructive treatments prior to SDS-PAGE and Western immunoblot analysis demonstrates that the antigen is susceptible to treatment with proteinase K. Accordingly, the protein nature of the antigen is shown because the reactivity of the antigen with YBY088 is destroyed by treatment with proteinase K.

(e) Isoelectric focusing of the antigen using two-dimensional gel electrophoresis indicates that the antigen has isoelectric points, with respect to each molecular weight component of the antigen noted above, within the ranges of about pH 8.0 to about pH 8.8, about pH 5.2 to about pH 5.8, about pH 6.2 to about pH 6.8, and about pH 8.0 to about pH 8.8. More specifically, the isoelectric peaks of the antigen are about pH 8.4, about pH 5.5, about pH 6.5, and about pH 8.4.

(f) The antigen is not detectable in human milk. By SDS-PAGE and Western immunoblot analysis, there is no detectable reactivity of YBY088 with human milk fat globule membranes.

(g) The antigen is not associated with blood components. By fluorescence activated cell sorter analysis (FACS), there is no detectable reactivity of YBY088 with blood components, including lymphocytes, red blood cells, myeloid components and platelets.

Summarizing the foregoing, the antigen of the present invention, which may be derived from a source selected from membrane or cytosol purified from normal human tissue or human tumor tissue, is characterized as being protein in nature and having molecular weight components within the ranges of about 68,000 to about 80,000, about 40,000 to about 48,000, about 28,000 to about 32,000, and about 16,000 to about 19,000. Additionally, the antigen is characterized as having isoelectric points, with respect to each molecular weight component of the antigen, within the ranges of about pH 8.0 to about pH 8.8, about pH 5.2 to about pH 5.8, about pH 6.2 to about pH 6.8, and about pH 8.0 to about pH 8.8. Further, the tumor-associated protein of the present invention is present in the cytosol of a variety of normal and tumor tissue and is expressed by the membrane of breast carcinoma.

Of particular importance in distinguishing the antigen of the present invention from other antigens, including other tumor-associated antigens, is the specificity of monoclonal antibody YBY088 for at least one determinant of the antigen. The specific reactivity of YBY088 for the antigen characterized by the invention provides a means for isolation and purification of the antigen from other material of human origin, and ultimately, characterization of the precise antigenic determinants comprising the antigen. Such a purified antigen and determinants thereof are useful in the production of monoclonal and polyclonal antibodies for diagnostic and therapeutic application using techniques well known to the art. For example, murine hybridomas producing monoclonal antibodies directed against the antigen may be obtained. Additionally, the antigen and determinants thereof may be used to generate monoclonal antibodies directed against molecules, other than the antigen of the present invention, having a specific determinant or determinants in common with the antigen characterized herein. This is particularly useful if certain antigens or determinants thereof are expressed differently during different stages or manifestations of a disease state. Alternatively, the antigen may be used to stimulate an immune response in a rabbit, goat or other animal from whose serum polyclonal antibodies may be obtained. Further, the antigen may be used for the characterization of antibodies of interest, e.g., monoclonal antibodies or antibodies present in human tissue, blood or other body fluids.

In accordance with the present invention, monoclonal antibodies having specificity for the antigen characterized herein and methods for their production, are provided. Preferably, such monoclonal antibodies are of the IgG class and possess an immunoreactivity with the tumor-associated protein of the present invention substantially similar to that of monoclonal antibody YBY088. Such monoclonal antibodies are useful in the detection, diagnosis and treatment of cancer in humans, particularly breast carcinoma. Further, such antibodies have application in the further isolation and purification of the antigen provided by the invention and the characterization of precise determinants.

Monoclonal antibodies as described above may be produced generally following the method of Kohler and Milstein, Nature 256, 495-497 (1975). In accordance with the present invention, a mouse or other suitable host is immunized with the soluble fraction of the purified antigen of the invention or the soluble fraction of human tumor tissue derived from a breast carcinoma. Following immunization, the spleen cells of the immunized mouse are fused with the cells from a suitable mouse myeloma line to obtain a mixture of hybrid cell lines. The hybrid cell lines are cultured in a suitable medium and, thereafter, hybrid cell lines producing an antibody having specific reactivity with the antigen characterized herein are selected and cloned, and monoclonal antibody thus produced is recovered.

Thus, in accordance with the invention, there is provided a process for preparing a monoclonal antibody which comprises:

(a) immunizing a host with the soluble fraction of human tumor tissue derived from a human breast carcinoma or an antigen derived from human tissue or a cancerous human cell line, said antigen being a protein having molecular weight components within the ranges of about 68,000 to about 80,000, about 40,000 to about 48,000, about 28,000 to about 32,000, and about 16,000 to about 19,000, and isoelectric points, with respect to said molecular weight components, within the ranges of about pH 8.0 to about pH 8.8, about pH 5.2 to about pH 5.8, about pH 6.2 to about pH 6.8, and about pH 8.0 to about pH 8.8.

(b) fusing spleen cells of said host with suitable myeloma cells to obtain a hybrid cell line;

(c) culturing said hybrid cell lines in a suitable medium and selecting and cloning those hybrid cell lines reactive to the antigen defined in (a); and

(d) recovering the monoclonal antibody produced.

The present invention suggests methods for the in vitro detection and diagnosis of cancer in humans, particularly breast carcinoma. Characterization of the unique tumor-associated protein of the invention as set forth herein, permits its detection in patient tissue samples by immunohistochemical methods and/or in patient fluid samples by in vitro immunoassay methods. A determination of the presence and/or quantity of the tumor-associated antigen of the present invention in patient specimens by immunohistochemical and/or immunoassay procedures is of significant diagnostic utility and may be indicative of or correlate with the progression of a disease state.

Immunohistochemical methods for the detection of antigens in patient tissue specimens are well known to the art and need not be described in detail. Briefly, in the context of the present invention, a tissue specimen obtained from a suspected cancer patient is contacted with an antibody, preferably a monoclonal antibody, having specificity for the tumor-associated protein characterized herein. Particularly preferred for use is monoclonal antibody YBY088. The sites at which the antibody is bound is thereafter determined by selective staining of the tissue specimen by standard immunohistochemical procedures.

Similarly, methods for the in vitro detection of antigenic substances in patient fluid samples by immunoassay procedures are well known to the art and require no repetition herein. For purposes of the present invention, a patient fluid sample is contacted with at least one antibody, preferably a monoclonal antibody, having specific reactivity with the tumor-associated protein of the invention and the antibody bound to sample components is determined. Qualitative and/or quantitative determinations of the presence of the antigen defined by the invention may be accomplished by competitive or non-competitive immunoassay procedures. Monoclonal antibodies or polyclonal antibodies may be utilized provided such antibodies possess the requisite specificity for the antigen provided by the present invention. Preferably, monoclonal antibody YBY088 is utilized. Additionally, the immunoassays preferred for use are two-site immunometric assays employing monoclonal antibodies which are selected to bind to non-interfering determinants of the antigen characterized herein.

The present invention further suggests methods for the in vivo diagnosis and therapy of cancer in humans, particularly breast carcinoma. Methods for tumor localization and detection may be performed, in accordance with the present invention, by administering to a suspected cancer patient a predetermined effective amount of an antibody having specific reactivity with the tumor-associated protein of the present invention, and thereafter, detecting the sites of localization of the antibody by standard imaging techniques. The antibody, preferably YBY088, is labeled, preferably with a radionuclide emitting gamma-radiation, so as to permit detection, and administered to the patient in a pharmaceutically acceptable carrier.

Thus, as an additional aspect of the invention, there is provided a therapeutic or diagnostic formulation which comprises a monoclonal antibody, as described, associated with a pharmaceutically- or diagnostically-acceptable carrier or diluent therefor. A pharmaceutically acceptable carrier is one useful in the treatment or diagnosis of a warm-blooded animal. A diagnostically-acceptable carrier is one which is useful in diagnostic testing procedures. Such carriers or diluents are completely familiar to one skilled in the art and require no further explanation.

In accordance with methods permitted by the present invention for cancer therapy, a predetermined effective amount of an antibody, preferably a monoclonal antibody, having specificity for the tumor-associated antigen characterized by the invention is administered to a cancer patient. The antibody, preferably YBY088, is conjugated with a suitable therapeutic agent, e.g., radioisotopes, preferably emitters of beta particles, drugs, toxins, or biological proteins selected for delivery to the tumor site, and administered to the cancer patient in a pharmaceutically-acceptable carrier.

Additionally, in the context of in vivo cancer diagnosis and therapy, those skilled in the art will appreciate that antibody preparations comprising mixtures of antibodies or fragments thereof, i.e., antibody cocktails, having specificity for the tumor-associated antigen of the invention may be used in certain instances to enhance the detection, localization and treatment of tumors.

The following non-limiting examples are provided to further illustrate the invention.

## EXAMPLE I

### Production of Monoclonal Antibody YBY088

Lymph node lymphocytes obtained from a patient diagnosed with breast cancer were thawed and cultured overnight in RPMI-1640 (Flow Laboratories, Alexandria, VA) containing 10% fetal bovine serum (PBS). $30 \times 10^6$ lymphocytes and $30 \times 10^6$ of P3/HT, (a subline of mouse myeloma cell line P3x63Ag8.653 (ATCC CRL 1580)) were fused with 35% polyethylene glycol-1000 in serum-free RPMI-7.5% dimethyl sulfoxide. Cells were plated out at densities of $10^5$ total cells per well in 96-well plates (Costar, Cambridge, MA) in RPMI-10% FBS - HAT $10^{-4}$ M hypoxanthine, $4 \times {}^-{}^7$ M aminopterin and $1.6 \times 10^{-5}$ M thymidine). Cultures were maintained for 2 weeks in 5% $CO_2$ and thereafter supernatant was replaced with fresh HAT media once per week. Supernatant from wells with growing cultures were sampled at day 40 and tested for the presence of Ig by enzyme-linked immunoabsorbent binding assay (ELISA).

Ig-producing clones were then screened by ELISA for specific reactivity with membranes and cytosols from breast tumors. The monoclonal antibody, an IgM antibody, was designated as YBY088 and further characterized and selected for use in the characterization of the antigen as provided herein.

## EXAMPLE II

### Antigen Characterization

Monoclonal antibody YBY088 was used to characterize the antigen of the present invention by the procedures set forth herein.

Membrane and cytosol fractions of human tissue were used for antigen characterization by screening the reactivity of monoclonal antibody YBY088 and were prepared as follows:

Surgical and autopsy specimens collected within ten hours after death were cut into blocks and stored at -80° C. Tissue was homogenized in four volumes of 10mM tris-HCl pH 7.5, 2mM calcium chloride, 2mM phenylmethylsulfonate (homogenization buffer) at 4° C. in a Dounce homogenizer. All subsequent steps were carried out at 4° C. The homogenate was centrifuged at $100 \times g$ for 5 minutes to remove nuclei and intact cells. The supernatant was removed and centrifuged at 130,000 x g for one hour. The supernatant from this high speed centrifugation was removed and designated as the cytosol fraction. The pellet was resuspended in one volume of homogenization buffer and layered on a 40%/20% discontinuous sucrose gradient in 10mM tris-HCl pH 7.2. The gradient was centrifuged at 130,000 x g for 17 hours. Material at the 40%/20% interphase was pipetted off, and diluted 5-fold in homogenization buffer and centrifuged for 60 minutes at 25,000 x g. The pellet was resuspended in homogenization buffer, aliquoted and stored at -80° C.

### Membrane/Cytosol ELISA

The presence of antigen reactive with monoclonal antibody YBY088 in purified membrane and cytosol fractions of normal human tissue and human tumor tissue was determined by an enzyme-linked immunoabsorbent binding assay (ELISA) as measured by absorbance at 490 nm.

To perform the membrane/cytosol ELISA, 10 $\mu$g/well of cytosol or 2 $\mu$g/well of membrane, prepared as described above, were dried onto flat-bottom microliter plates (Dynatech, Alexandria, GA) at 37° C. overnight. The plates were washed three times with cold tap water. 50 $\mu$l of 20% horse serum in PBS (phosphate buffered saline, 0.15M NaCl, 20mM sodium phosphate pH 7.2) were added followed by 50 $\mu$l of hybridoma supernatant and incubated for one hour at room temperature. After washing, the wells were incubated with 100 $\mu$l of a monoclonal mouse anti-human IgM-HRP conjugate (ZSAG11, Boehringer Mannheim, Indianapolis, IN, 1985/86 Catalog #0952, conjugated by the method of Wilson, M.B. & Nakane, P.K., Elsevier North Holland Biomedical Press, 1978) diluted 1:1000 in 5% horse serum-PBS for one hour. Bound enzyme was detected by incubation with 100 $\mu$l of 1 $\mu$g/ml o-phenylenediamine, 0.03% $H_2O_2$ in 0.1M citrate-phosphate buffer pH 5.0, for 30 minutes in the dark. Wells were quenched by the addition of 50 $\mu$l per well of $NH_4SO_4$. Absorbance at 490nM was read in a Dynatech ELISA plate reader. All assays included positive controls which routinely provided O.D. value of 1.0 to 2.0 and data by which to normalize among individual ELISA's.

As shown by Table I below, YBY088 was reactive with membrane fractions of breast carcinoma and cytosol fractions of a variety of normal tissues, including breast, colon, kidney, lung, liver, brain, prostrate and pancreas tissue. Accordingly, the presence of the antigen of the invention is indicated in the membrane of breast carcinoma and the cytosol of a number of benign tissues. As shown by Table I, the antigen is not associated with the membrane of other normal tissues, including colon, kidney, lung, liver, prostrate, brain and bladder. A relatively weak reactivity of YBY088, however, is shown with membrane fractions of normal breast and pancreas tissue and the mucosa of colon carcinoma indicating the presence of the antigen in the membrane of such tissues in relatively low concentrations.

## Biochemical Analysis of Antigen SDS-PAGE and Western Immunoblot Analysis

The molecular weight and biochemical nature of the antigen characterized by YBY088 were determined by SDS polyacrylamide gel electrophoresis (SDS-PAGE) and Western immunoblot analysis.

A human breast cell line, T47D, (R. Dulbecco, Salk Institute, ATCC HTB 133) was used in the examples to determine the molecular weight and biochemical nature of the antigen and was prepared as follows. T47D cell monolayers cultured in Autoclavable MEM (Irvine Scientific, Irvine California), supplemented with 8% horse serum-2% fetal bovine serum were rinsed three times in PBS, scraped from the flasks with a rubber policeman and washed in PBS by centrifugation. Washed cells were resuspended at approximately 20 x 10$^6$ cells per ml in one of the following buffers: (1) 0.5% NP40-0.5% deoxycholate-5mM EDTA-100mM Tris pH 8.0; and (2) 2% SDS-0.125M Tris pH 6.8. Cell suspensions were sonicated (Heat Systems, Ultrasonics Inc., model W-375), and airfuged (Beckman, Palo Alto, CA) at 130,000 x g for 30 minutes.

25 $\mu$g of T47D extracts, prepared as describecd above, were dissolved in gel sample buffer (0.12M Tris pH 6.8, 4% SDS, 20% glycerol, 0.002% bromophenol blue, 5% 2-mercaptoethanol) and separated by discontinuous SDS-page (Laemmli, U.K. Nature 227:680-685, 1970) on a 7.5% or 10% gel with a 3% stacking gel.

The separated proteins were electrophoretically transferred to nitrocellulose (0.1 m, Schleicher & Schuell, Inc., Keene, New Hampsire) at 200mA for three hours according to the method of Towbin et al., PNAS 76 43 (1979). The nitrocellulose replicas were then incubated with 3% bovine serum albumin (BSA)-PBS for 30 minutes, after which they were incubated with hybridoma supernatant diluted 1:1 with 5% skim milk-0.001% thimerosal-0.1% anti-foam A emulsion (Sigma Chemical Co., St. Louis, MO) overnight, in sealed pouches. The nitrocellulose strips were washed with PBS-0.1% Tween for 30 minutes with five changes of buffer, and incubated with $^{125}$I-labeled goat antihuman IgM (Tago, Burlingame, CA), 500,000 cpm/ml skim milk reagent, for two hours. After washing with PBS-0.1% Tween, the nitrocellulose strips were air-dried and exposed on X-ray film (Kodak XAR-5) for 18-72 hours with intensifying screen (Cronex DuPont Lightning Plus) at -70°.

To determine the biochemical nature of the antigen, the antigen was subjected to the following destructive treatments prior to SDS-PAGE and Western immunoblot analysis.

(a) Periodate oxidation of antigen prior to PAGE: Samples of the T47D cell line were adjusted to 50 mM $NaIO_4$ in PBS, pH 7.4 and incubated at 0° C. for one hour. Alternatively, sample was adjusted to 10mM $NaIO_4$ in 50 mM Na acetate, pH 4.5 and incubated at 0° C. for one hour.

(b) Periodate oxidation of antigen after fractionation by PAGE: Samples of the T47D cell line were first fractionated by PAGE and subsequently immobilized on nitrocellulose paper by Western blotting. The nitrocellulose strips were incubated in 10mM $NaIO_4$, 50 mM Na acetate, pH 4.5 for one hour at 4° C. Following incubation, the treated strips were incubated in 50 mM $NaBH_4$ for 30 minutes at room temperature and then washed three times with PBS prior to immunostaining.

(c) Mild alkali oxidation: To release O-linked carbohydrate from protein, samples of the T47D cell line were adjusted to 0.1 N NaOH and incubated for 18 hours at room temperature. The pH was brought to 7.4 with 1 N HCl prior to addition of sample buffer for electrophoresis. Alternately, antigen immobilized on nitrocellulose strips as in (b) was treated with 0.1 N NaOH at room temperature for 18 hours. The strips were washed three times with PBS-0.1% Tween prior to immunostaining.

(d) Neuraminidase digestion: Samples of the T47D cell line were adjusted to pH 5 with 100 mM HOAc and neuraminidase (Calbiochem) was added to a final concentration of 0.1 unit/ml. The sample was incubated one hour at 37° C. and the digestion was stopped by the addition of SDS-PAGE sample buffer followed by heating at 100° C. for five minutes.

(e) Endoglycosidase F Digestion: Endoglycosidase F was prepared by the method of Elder and Alexander (1982) PNAS 79:4540. Pooled fractions containing enzyme were stored in 50% glycerol at -20° C. A sample of the T47D cell line was diluted with 0.1 M phosphate buffer, pH 6.1 containing 0.05 M EDTA, 1% NP-40, 0.1% SDS, 1% 2-mercaptoethanol to a final volume of 40-80 μl. The sample was boiled for 2 minutes, cooled, and endo F was added (1:5 v:v). The sample was incubated overnight at 37° and the reaction was stopped by addition of SDS-PAGE sample buffer followed by heating at 100° C. for five minutes.

(f) Proteinase K digestion: Samples containing antigen were treated with proteinase K (Sigma, Type II) by the addition of 40 μg of enzyme/sample. Digestion was carried out at 37° for 1 hour and was terminated by the addition of SDS-PAGE sample buffer followed by heating at 100° C. for five minutes.

All reactions were stopped by adding gel sample buffer.

By SDS-PAGE and Western immunoblot analysis using SDS extracts the T47D breast cell line, the molecular weight components of the antigen were determined to be within the ranges of about 68,000 to about 80,000, about 40,000 to about 48,000, about 28,000 to about 32,000, and about 16,000 to 19,000. Alternatively, using non-ionic detergent extracts of the T47D breast cell line, the molecular weight components of the antigen were determined to be within the ranges of about 28,000 to about 32,000, and about 16,000 to about 19,000. Chemical and enzyme modification of the antigen folllowed by SDS-PAGE and Western immunoblotanalysis demonstrated the protein nature of the antigen. Specifically, reactivity of the antigen with YBY088 was shown to be destructible by treatment of the antigen with proteinase K. The loss of reactivity of the antigen with YBY088 did not result from the alternative modifications described above.

Isoelectric Focusing Two-Dimensional-Gel Electrophoresis

Two-dimensional gel analysis was performed using detergent or urea extracts of T47D cells, essentially according to the method of O'Farrell (O'Farrell, P.H., J. Biol. Chem. 250:4007-4021, 1975). Isoelectric focusing tube gels with pH 3.5-9.5 ampholines (LKB) were pre-focused for 15 minutes at 200 volts, 30 minutes at 300 volts and 30 minutes at 400 volts, followed by focusing at 300 volts for 16 hours. Second dimension separation on SDS-PAGE was carried out on 5-15% gels. Electroblotting to nitrocellulose and immunostaining analysis was then performed as described.

Isoelectric focusing of the antigen by this technique demonstrated isoelectric points, with respect to each molecular weight component of the antigen, to be within the ranges of about pH 8.0 to about pH 8.8, about pH 5.2 to about pH 5.8, about pH 6.2 to about pH 6.8, and about pH 8.0 to about pH 8.8.

Analysis of Antibody Reactivity With Milk Fat Globule Membranes

To determine whether the antigen characterized herein is detectable in human milk, the reactivity of YBY088 was evaluated with human milk fat globule membranes by standard procedures as described below.

Human milk fat globule membranes were prepared as follows. Human milk was centrifuged at 10,000 × g for 15 minutes. The cream, which floated to the top of the tube, was recovered and suspended in 0.01M tris-HCl buffer pH 7.5 containing 1mM MgCl$_2$ and 0.28M sucrose. This suspension was recentrifuged, and the washed cream was resuspended in 0.05M tris-HCl buffer pH 7.5. The washed milk fat globule membranes were homogenized for 10 minutes at 10,000 rpm using a Brinkman polytron. After incubation at 37° for 20 minutes which allowed the triglycerides to coalesce, the suspension was centrifuged at 105,000 × g for one hour at 37°. Milk fat globule membranes in the pellet were suspended in ice-cold 0.2M tris-HCl buffer pH 8.0 containing 1mM PMSF at a protein concentration of 10 mg/ml.

SDS-PAGE and Western immunoblot analysis were performed essentially as described above using 25 μg of human milk fat globule membranes, prepared as described above.

Analysis of Antibody Reactivity With Blood Components

To determine whether the antigen is associated with blood components, the reactivity of YBY088 was evaluated with blood components, including lymphocytes, red blood cells, myeloid centrifuged twice at 1000 rpm for 5-8 minutes each. From this, plasma was collected and centrifuged at 2000 rpm for 4-6 minutes to pellet the platelets which were resuspended in an appropriate volume of plasma. The buffy layer was also collected and centrifuged twice at 1000 rpm for 4-6 minutes each to collect lymphocytes. 10-12 ml of 0.83% ammonium chloride-0.14% potassium bicarbonate-0.05mM EDTA pH 7.3 was added to the pellet, incubated for 5 minutes and centrifuged again at 1000 rpm for 4-6 minutes. The pellet was washed once and resuspended in RPMI-10% fetal bovine serum at a concentration of 40 million cells/ml. Red blood cells were also resuspended in RPMI-10% fetal bovine serum at a concentration of 40 million cells/ml.

50 $\mu$l of the human antibody supernatant were dispensed into 96-well microtiter plates, followed by 25 $\mu$l (1 million cells) of cell suspension and incubated for 30 minutes at 4° C. Cells were washed three times with PBS by centrifugation and incubated with 50 $\mu$l of goat anti-human IgM-FITC conjugate (Tago, Burlingame, CA) for 30 minutes at 4° C. Cells were again washed as before, transferred to tubes containing 0.5-1.0 ml 1% formalin and subjected to analysis on a fluorescence activated cell sorter.

The above analysis indicated no specific reactivity of YBY088 with human milk fat globule membranes or blood components, including lymphocytes, red blood cells, myeloid components and platelets. Accordingly, the antigen characterized by the present invention is not detectable in human milk nor is the antigen associated with blood components.

## TABLE I
### YBY088 Reactivity with Normal Tissues and Tumor Tissues
#### ELISA Reactivity*

|  | Membrane | Cytosol |
|---|---|---|
| Breast Cancer #1 | 0 | 2 |
| Breast Cancer #2 | 170 | 2 |
| Breast Cancer #3 | 83 | 0 |
| Breast Cancer #4 | 66 | 1 |
| Breast Cancer #5 | 0 | 50 |
| Colon Cancer #1 | 0 | 24 |
| Colon Cancer #2 | 0 | 0 |
| Mucin Colon Cancer | 8 | 27 |
| Benign Breast #1 | 0 | 5 |
| Benign Breast #2 | 10 | 0 |
| Benign Colon | 0 | 26 |
| Benign Kidney | 0 | 22 |
| Benign Lung #1 | 0 | 2 |
| Benign Lung #2 | 0 | 55 |
| Benign Liver | 0 | 31 |
| Benign Brain | 0 | 26 |
| Benign Prostrate | 0 | 6 |
| Benign Pancreas | 38 | 30 |
| Benign Bladder | 0 | 0 |

*Expressed as percent (%)

$$\text{maximum control} = 100 \times \frac{\text{O.D.}_{490nm} \text{ test antibody}}{\text{O.D.}_{490nm} \text{ positive control antibody}}$$

**Claims**
**Claims for the following Contracting States : BE, CH, DE, FR, GB, IT, LI, LU, NL, SE**

1. An antigen derived from human tissue, said antigen being characterized as a protein having molecular weight components within the ranges of about 68,000 to about 80,000, about 40,000 to about 48,000, about 28,000 to about 32,000, and about 16,000 to about 19,000, and isoelectric points, with respect to said molecular weight components, within the ranges of about pH 8.0 to about pH 8.8, about pH 5.2 to about pH 5.8, about pH 6.2 to about pH 6.8, and about pH 8.0 to about pH 8.8, said antigen being further characterized by its reactivity with the monoclonal antibody YBY088 which is produced by the hybridoma cell-line ATCC HB9076.

2. An antigen as claimed in claim 1 wherein said antigen has isoelectric peaks at about pH 8.4, about pH 5.5, about pH 6.5, and about pH 8.4, respectively, for each molecular weight component.

3. An antigen as claimed in claim 2 in which the reactivity of said antigen with monoclonal antibody YBY088 is destructible by the treatment of said antigen with proteinase K.

4. An antigen as claimed in any one of claims 1 to 3 in which the associated antigen is a tumor associated antigen expressed by breast carcinoma.

5. An antibody having reactivity against an antigen as claimed in any one of claims 1 to 4.

6. An antibody as claimed in claim 5 which is a monoclonal antibody.

7. A monoclonal antibody as claimed in claim 6 which is reactive to a tumor-associated protein expressed by breast carcinoma.

8. A monoclonal antibody having specific reactivity with a tumor-associated protein having molecular weight components within the ranges of about 68,000 to about 80,000, about 40,000 to about 48,000, about 28,000 to about 32,000, and about 16,000 to about 19,000 and having isoelectric points, with respect to each molecular weight component within the ranges of about pH 8.0 to about pH 8.8, about pH 5.2 to about pH 5.8, about pH 6.2 to about pH 6.8, and about 8.0 to about pH 8.8, said protein being further characterized by its reactivity with the monoclonal antibody YBY088 which is produced by the hybridoma cell-line ATCC HB9076.

9. Monoclonal antibody YBY088.

10. Hybrid cell line ATCC HB9076.

11. An antibody, as claimed in any one of claims 5 to 9, for use as a diagnostic or therapeutic agent.

12. A diagnostic or therapeutic formulation which comprises an antibody, as claimed in any one of claims 5 to 9, associated with a diagnostically- or pharmaceutically-acceptable diluent therefor.

13. A method for the detection and diagnosis of cancer in a patient suspected of having cancer which comprises contacting a tissue specimen or fluid sample obtained from said patient with an antibody having specific reactivity with an antigen as defined in any one of claims 1 to 4.

**Claims for the following Contracting States : AT, GR**

1. A process for preparing a monoclonal antibody which comprises:
   (a) immunizing a host with the soluble fraction of human tumor tissue derived from a human breast carcinoma or an antigen derived from human tissue or a cancerous human cell line, said antigen being a protein having molecular weight components within the ranges of about 68,000 to about 80,000, about 40,000 to about 48,000, about 28,000 to about 32,000, and about 16,000 to about 19,000, and isoelectric points, with respect to said molecular weight components, within the ranges of about pH 8.0 to about pH 8.8, about pH 5.2 to about pH 5.8, about pH 6.2 to about pH 6.8, and about pH 8.0 to about pH 8.8, and said antigen being further characterized by its reactivity with the

10

monoclonal antibody YBY088 which is produced by the hybridoma cell-line ATCC HB9076;

(b) fusing spleen cells of said host with suitable myeloma cells to obtain a hybrid cell line;

(c) culturing said hybrid cell lines in a suitable medium and selecting and cloning those hybrid cell lines reactive to the antigen defined in (a); and

(d) recovering the monoclonal antibody produced.

2. A process as claimed in claim 1 in which the isoelectric peaks for the molecular weight components of about 68,000 to about 80,000, about 40,000 to about 48,000, about 28,000 to about 32,000, and about 16,000 to about 19,000 are about pH 8.4, about pH 5.5, about pH 6.5 and about pH 8.4, respectively.

3. A process as claimed in claim 2 in which the antigen - monoclonal antibody reactivity is destructible by treatment of the antigen with proteinase K.

4. A process as claimed in any one of claims 1 to 3 in which the antigen is a tumor-associated antigen expressed by breast carcinoma.

5. A process as claimed in any one of claims 1 to 4 in which the hybrid cell line is ATCC HB 9076.

6. A process as claimed in any one of claims 1 to 5 in which the monoclonal antibody produced is YBY088.

7. Monoclonal antibody YBY088.

8. Hybrid cell ATCC HB 9076.

9. A diagnostic or therapeutic formulation for the detection or treatment of cancer which comprises an antibody produced by the process of any one of claims 1 to 6, associated with a diagnostically- or pharmaceutically-acceptable diluent or carrier therefor.

10. A formulation as claimed in claim 9 which comprises monoclonal antibody YBY088.

11. The use of an antigen, as defined in any one of claims 1 to 4, for the manufacture of a composition for the treatment or detection of cancer.

12. The use of a monoclonal antibody, as defined in any one of claims 1 to 7, for the manufacture of a composition for the treatment or detection of cancer.

13. A method for the detection and diagnosis of cancer in a patient suspected of having cancer which comprises contacting a tissue specimen or fluid sample obtained from said patient with an antibody as defined in any one of claims 1 to 7.

14. An antigen as defined in any one of claims 1 to 4.

15. A monoclonal antibody which is reactive to an antigen as defined in any one of claims 1 to 4.

**Claims for the following Contracting State : ES**

1. A process for preparing a monoclonal antibody which comprises:

(a) immunizing a host with the soluble fraction of human tumor tissue derived from a human breast carcinoma or an antigen derived from human tissue or a cancerous human cell line, said antigen being a protein having molecular weight components within the ranges of about 68,000 to about 80,000, about 40,000 to about 48,000, about 28,000 to about 32,000, and about 16,000 to about 19,000, and isoelectric points, with respect to said molecular weight components, within the ranges of about pH 8.0 to about pH 8.8, about pH 5.2 to about pH 5.8, about pH 6.2 to about pH 6.8, and about pH 8.0 to about pH 8.8, and said antigen being further characterized by its reactivity with the monoclonal antibody YBY088 which is produced by the hybridoma cell-line ATCC HB9076;

(b) fusing spleen cells of said host with suitable myeloma cells to obtain a hybrid cell line;

(c) culturing said hybrid cell lines in a suitable medium and selecting and cloning those hybrid cell lines reactive to the antigen defined in (a); and
(d) recovering the monoclonal antibody produced.

2. A process as claimed in claim 1 in which the isoelectric peaks for the molecular weight components of about 68,000 to about 80,000, about 40,000 to about 48,000, about 28,000 to about 32,000, and about 16,000 to about 19,000 are about pH 8.4, about pH 5.5, about pH 6.5 and about pH 8.4, respectively.

3. A process as claimed in claim 2 in which the antigen - monoclonal antibody reactivity is destructible by treatment of the antigen with proteinase K.

4. A process as claimed in any one of claims 1 to 3 in which the antigen is a tumor-associated antigen expressed by breast carcinoma.

5. A process as claimed in any one of claims 1 to 5 in which the hybrid cell line is ATCC HB 9076.

6. A process as claimed in any one of claims 1 to 6 in which the monoclonal antibody produced is YBY088.

7. Monoclonal antibody YBY088.

8. Hybrid cell ATCC HB 9076.

9. A diagnostic or therapeutic formulation for the detection or treatment of cancer which comprises an antibody produced by the process of any one of claims 1 to 6, associated with a diagnostically- or pharmaceutically-acceptable diluent or carrier therefor.

10. A formulation as claimed in claim 9 which comprises monoclonal antibody YBY088.

11. The use of an antigen, as defined in any one of claims 1 to 5, for the manufacture of a composition for the treatment or detection of cancer.

12. The use of a monoclonal antibody, as defined in any one of claims 1 to 7, for the manufacture of a composition for the treatment or detection of cancer.

13. A method for the detection and diagnosis of cancer in a patient suspected of having cancer which comprises contacting a tissue specimen or fluid sample obtained from said patient with an antibody as defined in any one of claims 1 to 7.

**Patentansprüche**
**Patentansprüche für folgende Vertragsstaaten : BE, CH, DE, FR, GB, IT, LI, LU, NL, SE**

1. Aus Humangewebe stammendes Antigen, dadurch gekennzeichnet, daß dieses Antigen ein Protein mit Molekulargewichtskomponenten in den Bereichen von etwa 68 000 bis etwa 80 000, etwa 40 000 bis etwa 48 000, etwa 28 000 bis etwa 32 000 und etwa 16 000 bis etwa 19 000 ist, daß die isoelektrischen Punkte bezüglich dieser Molekulargewichtskomponenten in den Bereichen von etwa pH 8,0 bis etwa pH 8,8, etwa pH 5,2 bis etwa pH 5,8, etwa pH 6,2 bis etwa pH 6,8 und etwa pH 8,0 bis etwa pH 8,8 liegen und daß dieses Antigen eine Reaktivität mit dem monoklonalen Antikörper YBY088 aufweist, der von der Hybridomazellinie ATCC HB9076 gebildet wird.

2. Antigen nach Anspruch 1, wobei das Antigen bezüglich dieser Molekulargewichtskomponenten isoelektrische Maximalwerte von etwa pH 8,4, etwa pH 5,5, etwa pH 6,5 und etwa pH 8,4 aufweist.

3. Antigen nach Anspruch 2, wobei die Reaktivität dieses Antigens mit dem monoklonalen Antikörper YBY088 durch die Behandlung dieses Antigens mit Proteinase K zerstört werden kann.

4. Antigen nach einem der Ansprüche 1 bis 3, wobei das assoziierte Antigen ein tumorassoziiertes Antigen ist, das von Brustkarzinomen exprimiert wird.

EP 0 242 154 B1

**5.** Antikörper, der eine Reaktivität gegen ein Antigen nach einem der Ansprüche 1 bis 4 aufweist.

**6.** Antikörper nach Anspruch 5, der ein monoklonaler Antikörper ist.

**7.** Monoklonaler Antikörper nach Anspruch 6, der mit einem von Brustkarzinomen exprimierten tumorassoziierten Protein reagiert.

**8.** Monoklonaler Antikörper, dadurch gekennzeichnet, daß dieser eine spezifische Reaktivität mit einem tumorassoziierten Protein besitzt, das Molekulargewichtskomponenten in den Bereichen von etwa 68 000 bis etwa 80 000, etwa 40 000 bis etwa 48 000, etwa 28 000 bis etwa 32 000 und etwa 16 000 bis etwa 19 000 aufweist und die isoelektrischen Punkte bezüglich dieser Molekulargewichtskomponenten in den Bereichen von etwa pH 8,0 bis etwa pH 8,8, etwa pH 5,2 bis etwa pH 5,8, etwa pH 6,2 bis etwa pH 6,8 und etwa pH 8,0 bis etwa pH 8,8 liegen und daß dieses Antigen eine Reaktivität mit dem monoklonalen Antikörper YBY088 aufweist, der von der Hybridomazellinie ATCC HB9076 gebildet wird.

**9.** Monoklonaler Antikörper YBY088.

**10.** Hybridzellinie ATCC HB9076.

**11.** Antikörper nach einem der Ansprüche 5 bis 9 zur Verwendung als diagnostisches oder therapeutisches Mittel eingesetzt wird.

**12.** Diagnostische oder therapeutische Formulierung, dadurch gekennzeichnet, daß sie einen Antikörper nach einem der Ansprüche 5 bis 9 zusammen mit einem diagnostisch oder pharmazeutisch annehmbaren Verdünnungsmittel hierfür enthält.

**13.** Verfahren zur Detektion und Diagnose von Krebs in einem Patienten mit Krebsverdacht, dadurch gekennzeichnet, daß eine vom Patienten entnommene Gewebsprobe oder Flüssigkeitsprobe mit einem Antikörper zusammengebracht wird, der eine spezifische Reaktivität mit einem Antigen gemäß einem der Ansprüche 1 bis 4 aufweist.

**Patentansprüche für folgende Vertragsstaaten : AT, GR**

**1.** Verfahren zur Herstellung eines monoklonalen Antikörpers, gekennzeichnet durch:
(a) Immunisieren eines Wirts mit der löslichen Fraktion von humanem Tumorgewebe, das von einem humanem Brustkarzinom stammt oder mit einem Antigen, das aus humanem Gewebe stammt oder mit einer krebsartigen Humanzellinie, wobei das Antigen ein Protein mit mit Molekulargewichtskomponenten in den Bereichen von etwa 68 000 bis etwa 80 000, etwa 40 000 bis etwa 48 000, etwa 28 000 bis etwa 32 000 und etwa 16 000 bis etwa 19 000 ist, die isoelektrischen Punkte bezüglich dieser Molekulargewichtskomponenten in den Bereichen von etwa pH 8,0 bis etwa pH 8,8, etwa pH 5,2 bis etwa pH 5,8, etwa pH 6,2 bis etwa pH 6,8 und etwa pH 8,0 bis etwa pH 8,8 liegen, und dieses Antigen eine Reaktivität mit dem monoklonalen Antikörper YBY088 aufweist, der von der Hybridomazellinie ATCC HB9076 gebildet wird.
(b) Fusionieren von Milzzellen des Wirts mit geeigneten Myelomzellen, um eine Hybridzellinie zu erhalten.
(c) Kultivieren der Hybridzellinie in einem geeigneten Medium und Selektieren und Klonieren jener Hybridzellinien, die gegen das in (a) definierte Antigen reaktiv sind und
(d) Gewinnung des gebildeten monoklonalen Antikörpers.

**2.** Verfahren nach Anspruch 1, worin die isoelektrischen Maximalwerte der Molekulargewichtskomponenten von etwa 68 000 bis etwa 80 000, etwa 40 000 bis etwa 48 000, etwa 28 000 bis etwa 32 000 und etwa 16 000 bis etwa 19 000 bei etwa pH 8,4, etwa pH 5,5, etwa pH 6,5 und etwa pH 8,4 liegen.

**3.** Verfahren nach Anspruch 2, worin die Antigen-Antikörper Reaktivität durch die Behandlung des Antigens mit Proteinase K zerstört werden kann.

**4.** Verfahren nach einem der Ansprüche 1 bis 3, worin das Antigen ein tumorassoziiertes Antigen ist, das von Brustkarzinomen exprimiert wird.

13

EP 0 242 154 B1

**5.** Verfahren nach einem der Ansprüche 1 bis 4, worin die Hybridzellinie ATCC HB 9076 ist.

**6.** Verfahren nach einem der Ansprüche 1 bis 5, worin der gebildete monoklonale Antikörper YBY088 ist.

**7.** Monoklonaler Antikörper YBY088.

**8.** Hybridzellinie ATCC HB 9076.

**9.** Diagnostische oder therapeutische Formulierung zur Detektion oder Behandlung von Krebs, dadurch gekennzeichnet, daß diese einen nach einem Verfahren der Ansprüche 1 bis 6 hergestellten Antikörper zusammen mit einem diagnostisch oder pharmazeutisch annehmbaren Verdünnungsmittel oder Träger hierfür enthält.

**10.** Formulierung nach Anspruch 9, die den monoklonalen Antikörper YBY088 enthält.

**11.** Verwendung eines in einem der Ansprüche 1 bis 4 definierten Antigens zur Herstellung einer Zusammensetzung für die Behandlung oder Detektion von Krebs.

**12.** Verwendung eines in einem der Ansprüche 1 bis 7 definierten monoklonalen Antikörpers zur Herstellung einer Zusammensetzung für die Behandlung oder Detektion von Krebs.

**13.** Verfahren zur Detektion und Diagnose von Krebs bei einem Patienten mit Krebsverdacht, dadurch gekennzeichnet, daß eine von diesem Patienten entnommene Gewebsprobe oder Flüssigkeitsprobe mit einem in einem der Ansprüche 1 bis 7 definierten Antikörper zusammengebracht wird.

**14.** Antigen, wie in einem der Anpsrüche 1 bis 4 definiert.

**15.** Monoklonaler Antikörper, der mit einem Antigen, wie in einem der Ansprüche 1 bis 4 definiert, reagiert.

**Patentansprüche für folgenden Vertragsstaat : ES**

**1.** Verfahren zur Herstellung eines monoklonalen Antikörpers, gekennzeichnet durch:
   (a) Immunisieren eines Wirts mit der löslichen Fraktion von humanem Tumorgewebe, das von einem humanem Brustkarzinom stammt oder mit einem Antigen, das aus humanem Gewebe stammt oder mit einer krebsartigen Humanzellinie, wobei das Antigen ein Protein mit mit Molekulargewichtskomponenten in den Bereichen von etwa 68 000 bis etwa 80 000, etwa 40 000 bis etwa 48 000, etwa 28 000 bis etwa 32 000 und etwa 16 000 bis etwa 19 000 ist, die isoelektrischen Punkte bezüglich dieser Molekulargewichtskomponenten in den Bereichen von etwa pH 8,0 bis etwa pH 8,8, etwa pH 5,2 bis etwa pH 5,8, etwa pH 6,2 bis etwa pH 6,8 und etwa pH 8,0 bis etwa pH 8,8 liegen, und dieses Antigen eine Reaktivität mit dem monoklonalen Antikörper YBY088 aufweist, der von der Hybridomazellinie ATCC HB9076 gebildet wird.
   (b) Fusionieren von Milzzellen des Wirts mit geeigneten Myelomzellen, um eine Hybridzellinie zu erhalten.
   (c) Kultivieren der Hybridzellinie in einem geeigneten Medium und Selektieren und Klonieren jener Hybridzellinien, die gegen das in (a) definierte Antigen reaktiv sind und
   (d) Gewinnung des gebildeten monoklonalen Antikörpers.

**2.** Verfahren nach Anspruch 1, worin die isoelektrischen Maximalwerte der Molekulargewichtskomponenten von etwa 68 000 bis etwa 80 000, etwa 40 000 bis etwa 48 000, etwa 28 000 bis etwa 32 000 und etwa 16 000 bis etwa 19 000 bei etwa pH 8,4, etwa pH 5,5, etwa pH 6,5 und etwa pH 8,4 liegen.

**3.** Verfahren nach Anspruch 2, worin die Antigen-Antikörper Reaktivität durch die Behandlung des Antigens mit Proteinase K zerstört werden kann.

**4.** Verfahren nach einem der Ansprüche 1 bis 3, worin das Antigen ein tumorassoziiertes Antigen ist, das von Brustkarzinomen exprimiert wird.

**5.** Verfahren nach einem der Ansprüche 1 bis 4, worin die Hybridzellinie ATCC HB 9076 ist.

14

**6.** Verfahren nach einem der Ansprüche 1 bis 5, worin der gebildete monoklonale Antikörper YBY088 ist.

**7.** Monoklonaler Antikörper YBY088.

**8.** Hybridzellinie ATCC HB 9076.

**9.** Diagnostische oder therapeutische Formulierung zur Detektion oder Behandlung von Krebs, dadurch gekennzeichnet, daß diese einen nach einem Verfahren der Ansprüche 1 bis 6 hergestellten Antikörper zusammen mit einem diagnostisch oder pharmazeutisch annehmbaren Verdünnungsmittel oder Träger hierfür enthält.

**10.** Formulierung nach Anspruch 9, die den monoklonalen Antikörper YBY088 enthält.

**11.** Verwendung eines in einem der Ansprüche 1 bis 4 definierten Antigens zur Herstellung einer Zusammensetzung für die Behandlung oder Detektion von Krebs.

**12.** Verwendung eines in einem der Ansprüche 1 bis 7 definierten monoklonalen Antikörpers zur Herstellung einer Zusammensetzung für die Behandlung oder Detektion von Krebs.

**13.** Verfahren zur Detektion und Diagnose von Krebs bei einem Patienten mit Krebsverdacht, dadurch gekennzeichnet, daß eine von diesem Patienten entnommene Gewebsprobe oder Flüssigkeitsprobe mit einem in einem der Ansprüche 1 bis 7 definierten Antikörper zusammengebracht wird.

**Revendications**

**Revendications pour les Etats contractants suivants : BE, CH, DE, FR, GB, IT, LI, LU, NL, SE**

**1.** Antigène dérivé de tissus humain, l'antigène étant caractérisé en tant qu'une protéine ayant des composés de poids moléculaire endéans les fourchettes d'environ 68.000 à environ 80.000, d'environ 40.000 à environ 48.000, d'environ 28.000 à environ 32.000 et d'environ 16.000 à environ 19.000, et des points isoélectriques, en relation avec les composés de poids moléculaire, endéans les fourchettes d'environ pH 8,0 à environ pH 8,8, d'environ pH 5,2 à environ pH 5,8, d'environ pH 6,2 à environ pH 6,8 et d'environ pH 8,0 à environ pH 8,8, ledit antigène étant caractérisé en plus par sa réactivité avec l'anticorps monoclonal YBY088 qui est produit par la lignée cellulaire hybridome ATCC HB9076.

**2.** Antigène tel que revendiqué dans la revendication 1, dans lequel ledit antigène a des maximums isoélectriques à environ pH 8,4, à environ pH 5,5, à environ pH 6,5, et à environ pH 8,4, respectivement, pour chaque composé de poids moléculaire.

**3.** Antigène tel que revendiqué dans la revendication 2, dans lequel la réactivité dudit antigène avec l'anticorps monoclonal YBY088 est destructible par le traitement dudit antigène avec la protéinase K.

**4.** Antigène tel que revendiqué dans l'une quelconque des revendications 1 à 3, dans lesquelles l'antigène associé est un antigène associé à une tumeur exprimée par un carcinome du sein.

**5.** Anticorps ayant une réactivité contre un antigène tel que revendiqué dans l'une quelconque des revendications 1 à 4.

**6.** Anticorps tel que revendiqué dans la revendication 5 qui est un anticorps monoclonal.

**7.** Anticorps monoclonal tel que revendiqué dans la revendication 6 qui réagit avec une protéine associée à une tumeur exprimée par le carcinome du sein.

**8.** Anticorps monoclonal ayant une réactivité spécifique avec des protéines associées à des tumeurs ayant des composés de poids moléculaire endéans les fourchettes d'environ 68.000 à environ 80.000, d'environ 40.000 à environ 48.000, d'environ 28.000 à environ 32.000, et d'environ 16.000 à environ 19.000 et ayant des points isoélectriques, en relation avec chaque composé de poids moléculaire endéans les fourchettes d'environ pH 8,0 à environ pH 8,8, d'environ pH 5,2 à environ pH 5,8, d'environ pH 6,2 à environ pH 6,8, et d'environ pH 8,0 à environ pH 8,8, ladite protéine étant de plus

15

caractérisée par sa réactivité avec l'anticorps monoclonal YBY088 qui est produit par la lignée cellulaire d'hybridome ATCC HB9076.

9.  Anticorps monoclonal YBY088.

10.  Lignée cellulaire hybride ATCC HB9076.

11.  Anticorps, tel que revendiqué dans l'une quelconque des revendications 5 à 9, pour l'utilisation en tant qu'agent de diagnose ou de thérapie.

12.  Formulation de diagnostic ou de thérapie qui comprend un anticorps, tel que revendiqué dans l'une quelconque des revendications 5 à 9, associé avec un diluant diagnostiquement ou pharmaceutiquement acceptable de celui-ci.

13.  Méthode pour la détection et le diagnostic du cancer chez un patient chez lequel on suspecte un cancer qui comprend la mise en contact d'un spécimen de tissus ou d'un échantillon de fluide obtenu dudit patient avec un anticorps ayant une réactivité spécifique avec l'antigène tel que défini dans l'une quelconque des revendications 1 à 4.

**Revendications pour les Etats contractants suivants : AT, GR**

1.  Procédé pour la préparation d'un anticorps monoclonal comprenant:
    (a) l'immunisation d'un hôte avec une fraction soluble d'un tissu de tumeur humaine dérivé d'un carcinome du sein humain ou un antigène dérivé d'un tissu humain ou une lignée cellulaire humaine cancéreuse, ledit antigène étant une protéine ayant des composés de poids moléculaire endéans les fourchettes d'environ 68.000 à environ 80.000, d'environ 40.000 à environ 48.000, d'environ 28.000 à environ 32.000, et d'environ 16.000 à environ 19.000, et des points isoélectriques en relation avec lesdits composés de poids moléculaire, dans les fourchettes d'environ pH 8,0 à environ pH 8,8, d'environ pH 5,2 à environ pH 5,8, d'environ pH 6,2 à environ pH 6,8, et d'environ pH 8,0 à environ pH 8,8, ledit antigène étant de plus caractérisé par sa réactivité avec l'anticorps monoclonal YBY088 qui est produit par la lignée cellulaire hybridome ATCC HB9076;
    (b) la fusion des cellules de la rate dudit hôte avec des cellules myélomiques adéquates pour obtenir une lignée de cellules hybrides;
    (c) la culture desdites lignées cellulaires hybrides dans des milieux adéquats et la sélection et le clonage des lignées de cellules hybrides reagissant avec l'antigène défini dans (a); et
    (d) la récupération de l'anticorps monoclonal produit.

2.  Procédé tel que revendiqué dans la revendication 1, dans laquelle les maxima isoélectriques pour les composés de poids moléculaire d'environ 68.000 à environ 80.000, d'environ 40.000 à environ 48.000, d'environ 28.000 à environ 32.000, d'environ 16.000 à environ 19.000 sont d'environ pH 8,4, d'environ pH 5,5, d'environ pH 6,5, et d'environ pH 8,4, respectivement.

3.  Procédé tel que revendiqué dans la revendication 2, dans laquelle la réactivité antigène-anticorps monoclonal est destructible par le traitement de l'antigène avec la protéinase K.

4.  Procédé tel que revendiqué dans l'une quelconque des revendications 1 à 3, dans lequel l'antigène est un antigène associé à une tumeur exprimée par un carcinome du sein.

5.  Procédé tel que revendiqué dans l'une quelconque des revendications 1 à 4, dans lequel la lignée de cellules hybrides est ATCC HB9076.

6.  Procédé tel que revendiqué dans l'une quelconque des revendications 1 à 5, dans lequel l'anticorps monoclonal produit est YBY088.

7.  Anticorps monoclonal YBY088.

8.  Cellule hybride ATCC HB9076.

9. Formulation diagnostique ou thérapeutique pour la détection ou le traitement de cancer qui comprend un anticorps produit par le procédé de l'une quelconque des revendications 1 à 6, associé avec un diluant ou un excipient de celui-ci diagnostiquement ou pharmaceutiquement acceptable.

10. Formulation telle que revendiquée dans la revendication 9 qui comprend un anticorps monoclonal YBY088.

11. Utilisation d'un antigène, tel que définie dans l'une quelconque des revendications 1 à 4, pour la fabrication d'une composition pour le traitement ou la détection de cancer.

12. Utilisation d'un anticorps monoclonal, tel que définie dans l'une quelconque des revendications 1 à 7, pour la fabrication d'une composition pour le traitement ou la détection de cancer.

13. Méthode pour la détection et la diagnose de cancer chez un patient chez lequel on suspecte un cancer qui comprend la mise en contact d'un spécimen de tissus ou d'un échantillon de fluide obtenu dudit patient avec un anticorps tel que défini dans l'une quelconque des revendications 1 à 7.

14. Antigène tel que défini dans l'une quelconque des revendications 1 à 4.

15. Anticorps monoclonal qui réagit avec un antigène tel que défini dans l'une quelconque des revendications 1 à 4.

**Revendications pour l'Etat contractant suivant : ES**

1. Procédé pour la préparation d'un anticorps monoclonal comprenant:
   (a) l'immunisation d'un hôte avec une fraction soluble d'un tissu de tumeur humaine dérivé d'un carcinome du sein humain ou un antigène dérivé d'un tissu humain ou une lignée cellulaire humaine cancéreuse, ledit antigène étant une protéine ayant des composés de poids moléculaire endéans les fourchettes d'environ 68.000 à environ 80.000, d'environ 40.000 à environ 48.000, d'environ 28.000 à environ 32.000, et d'environ 16.000 à environ 19.000, et des points isoélectriques en relation avec lesdits composés de poids moléculaire, dans les fourchettes d'environ pH 8,0 à environ pH 8,8, d'environ pH 5,2 à environ pH 5,8, d'environ pH 6,2 à environ pH 6,8, et d'environ pH 8,0 à environ pH 8,8, ledit antigène étant de plus caractérisé par sa réactivité avec l'anticorps monoclonal YBY088 qui est produit par la lignée cellulaire hybridome ATCC HB9076;
   (b) la fusion des cellules de la rate dudit hôte avec des cellules myélomiques adéquates pour obtenir une lignée de cellules hybrides;
   (c) la culture desdites lignées cellulaires hybrides dans des milieux adéquats et sélectionnant et clonant les lignées de cellules hybrides reagissant avec l'antigène défini dans (a); et
   (d) la récupération de l'anticorps monoclonal produit.

2. Procédé tel que revendiqué dans la revendication 1, dans laquelle les maxima isoélectriques pour les composés de poids moléculaire d'environ 68.000 à environ 80.000, d'environ 40.000 à environ 48.000, d'environ 28.000 à environ 32.000, d'environ 16.000 à environ 19.000 sont d'environ pH 8,4, d'environ pH 5,5, d'environ pH 6,5, et d'environ pH 8,4, respectivement.

3. Procédé tel que revendiqué dans la revendication 2, dans laquelle la réactivité antigène-anticorps monoclonal est destructible par le traitement de l'antigène avec la protéinase K.

4. Procédé tel que revendiqué dans l'une quelconque des revendications 1 à 3, dans lequel l'antigène est un antigène associé à une tumeur exprimée par un carcinome du sein.

5. Procédé tel que revendiqué dans l'une quelconque des revendications 1 à 4, dans lequel la lignée de cellules hybrides est ATCC HB9076.

6. Procédé tel que revendiqué dans l'une quelconque des revendications 1 à 5, dans lequel l'anticorps monoclonal produit et YBY088.

7. Anticorps monoclonal YBY088.

**8.** Cellule hybride ATCC HB9076.

**9.** Formulation diagnostique ou thérapeutique pour la détection ou le traitement de cancer qui comprend un anticorps produit par le procédé de l'une quelconque des revendications 1 à 6, associé avec un diluant acceptable ou un excipient de celui-ci diagnostiquement ou pharmaceutiquement.

**10.** Formulation telle que revendiquée dans la revendication 9 qui comprend un anticorps monoclonal YBY088.

**11.** Utilisation d'un antigène, tel que défini dans l'une quelconque des revendications 1 à 5, pour la fabrication d'une composition pour le traitement ou la détection de cancer.

**12.** Utilisation d'un anticorps monoclonal, tel que défini dans l'une quelconque des revendications 1 à 7, pour la fabrication d'une composition pour le traitement ou la détection de cancer.

**13.** Méthode pour la détection et la diagnose de cancer chez un patient chez lequel on suspecte un cancer qui comprend la mise en contact d'un spécimen de tissus ou d'un échantillon de fluide obtenu dudit patient avec un anticorps tel que défini dans l'une quelconque des revendications 1 à 7.